# EUROPEAN PATENT APPLICATION

(11) **EP 2 524 921 A1**
(43) Date of publication of application: **21.11.2012**
(21) Application number: 11166312.6
(22) Date of filing: 17.05.2011
(51) Int. Cl.: C07D 491/044, A61K 31/407, A61P 25/18

(54) **Novel Crystalline Salts of Asenapine**

(71) Applicant: Sandoz AG, 4056 Basel (CH)
(72) Inventor: Blatter, Fritz, 4153 Reinach (CH); Reichenbächer, Katharina, 4125 Riehen (CH)
(74) Representative: Pommerenke, Alexander

(57) **Abstract**

The present invention relates to novel crystalline salts of Asenapine and to methods of their preparation. In particular, the invention relates to crystalline Asenapine salts of tartaric acid or succinic acid, wherein the salts are e.g. selected from the group consisting of Asenapine D, L-tartrate hydrate form A, Asenapine D, L-tartrate hydrate form B, Asenapine D, L-tartrate anhydrous form , Asenapine succinate form X, Asenapine succinate form , and Asenapine succinate form II.
Furthermore the present invention relates to pharmaceutical compositions comprising the novel salts and to the novel salts for use in the treatment of psychotic diseases or disorders.

## Description

### Field of the invention

The present invention relates to novel crystalline salts of Asenapine and to methods of their preparation. Furthermore the present invention relates to the use of the novel salts in pharmaceutical compositions and the use of the novel salts in the treatment of psychotic diseases or disorders such as schizophrenia and acute mania associated with bipolar disorder.

### Background prior art

Asenapine, trademark Saphris®, chemically trans-5-chloro-2-methyl-2,3,3a,12b-tetrahydro-1 H-dibenz[2,3:6,7] oxepino[4,5-c]pyrrole, in sublingual dissolving tablet form, has been approved in US in August 2009 for the acute treatment of adult patients with schizophrenia and as monotherapy for acute mania or mixed episodes associated with bipolar disorders. The FDA has recently approved its use as ongoing maintenance treatment for schizophrenia and as adjunctive therapy with lithium or with valproate for bipolar 1 disorder.

Certain crystalline addition salts of Asenapine, for instance a fumarate (EP 0569096), salts with sulfonic acids (WO98/54186), and a pamoate or hemipamoate salt (EP 0569096), are described in the literature. The pamoate salt is disclosed to be amorphous and the hemipamoate salt is a mixture of amorphous and crystalline phase, wherein the palmitate is described as oil. The marketed form is the maleate salt, which is disclosed to exist in polymorphic forms (WO 2006/106135). The known Asenapine salts have a low solubility in water. For example, Funke et. al. (Arzneim.-Forsch./Drug Res. 40, 1999, 536-539) reports that a saturated solution of the maleate salt of Asenapine at 23°C has a concentration of 5.8 mg/ml at pH = 4.4. This translates into a free base solubility of about 4.1 mg/ml.

The discovery of new salts of a pharmaceutically useful compound provides a new opportunity to improve the performance characteristics of a pharmaceutical product. It enlarges the repertoire of materials that a formulation scientist has available for designing, for example a pharmaceutical dosage form of a drug with targeted release profile or other desired characteristic.

For example, the premise for a sublingual dissolving tablet form is an active ingredient exhibiting good solubility in a fast dissolving matrix.

It is an objective of the present invention to provide pharmaceutically acceptable salts of Asenapine with excellent solubility.

It is also an object of the present invention to provide Asenapine in form of a salt having good properties, in particular a good chemical and/or physical stability and/or good processability, both during its preparation and in the preparation of pharmaceutical compositions containing Asenapine salts.

The problems underlying the present invention are solved by the subject matters defined in the claims.

Surprisingly, it was found that crystalline salts of Asenapine according to formula I as described below may provide beneficial properties e.g. regarding solubility and may furthermore enhance the performance of dosage forms comprising said Asenapine salts. In particular, using tartaric acid or succinic acid for preparing Asenapine salts allows providing crystalline salts that may have enhanced properties.

It has additionally, been found in the context of the present invention that Asenapine hydrates of tartaric acid/succinic acid can be prepared by treating Asenapine salts in anhydrous form with water. It is furthermore possible to convert Asenapine hydrates having a particular crystal form into hydrates having a different crystal form upon treatment with water.

### Summary of the invention

In particular, the present invention refers to the following embodiments:
(1) A crystalline salt of Asenapine, preferably a hydrate, with an organic acid according to formula I 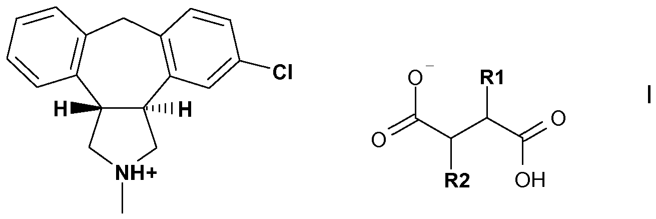 wherein R1 and R2 are the same and each represent H or OH.
(2) The crystalline salt of item (1) having a solubility in water of not less than 10 mg/ml calculated as free base equivalent, and/or wherein the ratio of Asenapine to organic acid in said salt is preferably about 1:1.
(3) The crystalline salt of item (1) or (2), wherein the organic acid represents D, L-tartaric acid or succinic acid and/or wherein said crystalline salt is a hydrate or is in anhydrous form, preferably the salt is a hydrate.
(4) The crystalline salt of any of items (1)-(4), wherein the salt is selected from the group consisting of Asenapine D, L-tartrate hydrate form A, Asenapine D, L-tartrate hydrate form B, Asenapine D, L-tartrate anhydrous form I, Asenapine succinate form X, Asenapine succinate form I, and Asenapine succinate form II, wherein
   Asenapine D, L-tartrate hydrate form A is characterized by X-ray powder diffraction reflections comprising peaks at about 18.5° ± 0.2°, 24.4° ± 0.2°, 17.4° ± 0.2°, 13.3° ± 0.2°, 15.0° ± 0.2°, and 6.6° ± 0.2° degrees two-theta;
   Asenapine D, L-tartrate hydrate form B characterized by X-ray powder diffraction reflections (Cu Kα radiation) comprising peaks at about 26.5°± 0.2°, 15.8°± 0.2°, 7.3°± 0.2°, 10.5°± 0.2°, 14.6°± 0.2°, 20.3°± 0.2°, and 23.5°± 0.2° degrees two-theta;
   Asenapine D, L-tartrate anhydrous form I is characterized by X-ray powder diffraction reflections (Cu Kα radiation) comprising peaks at about 17.5°± 0.2°, 13.1°± 0.2°, 4.4°± 0.2°, 30.8° ± 0.2°, and 16.9°± 0.2° degrees two-theta;
   Asenapine succinate form X is characterized by X-ray powder diffraction reflections (Cu Kα radiation) comprising peaks at about 22.6°± 0.2°, 21.5°± 0.2°, 10.8°± 0.2°, 21.0°± 0.2°, and 24.3° ± 0.2° degrees two-theta;
   Asenapine succinate form I is characterized by X-ray powder diffraction reflections (Cu Kα radiation) comprising peaks at about 21.1°± 0.2°, 22.0°± 0.2°, 10.5°± 0.2°, 16.5°± 0.2°, and 23.0° ± 0.2° degrees two-theta; and
   Asenapine succinate form II is characterized by X-ray powder diffraction reflections (Cu Kα radiation) comprising peaks at about 21.2°± 0.2°, 20.7°± 0.2°, 10.6°± 0.2°, 22.7° ± 0.2°, and 16.1 °± 0.2°, degrees two-theta.

Further preferred, the salt is selected from the group consisting of Asenapine D, L-tartrate hydrate form A, Asenapine D, L-tartrate hydrate form B, Asenapine succinate form X, Asenapine succinate form I, and Asenapine succinate form II. Also preferred, the salt is Asenapine D, L-tartrate hydrate form A or Asenapine succinate form X. Preferred hydrates are: Asenapine D, L-tartrate hydrate form A, Asenapine D, L-tartrate hydrate form B, Asenapine succinate form X, Asenapine succinate form I, and Asenapine succinate form II.

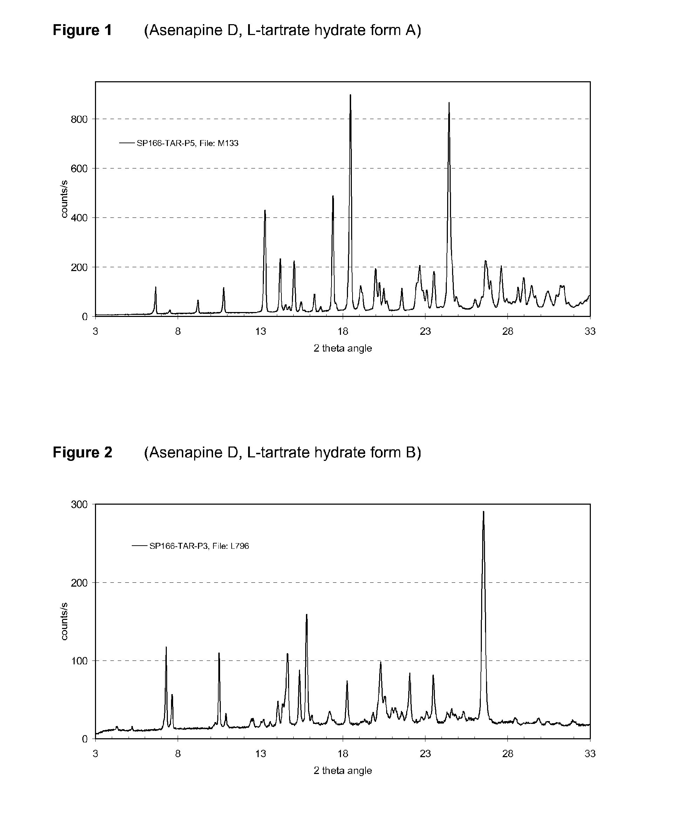

The crystalline Asenapine D, L-tartrate hydrate form A is preferably characterized by a PXRD pattern substantially in accordance with Figure 1, in particular the PXRD pattern comprises the peaks as given in Table 4.

The crystalline Asenapine D, L-tartrate hydrate form B is preferably characterized by a PXRD pattern substantially in accordance with Figure 2, in particular the PXRD pattern comprises the peaks as given in Table 3.

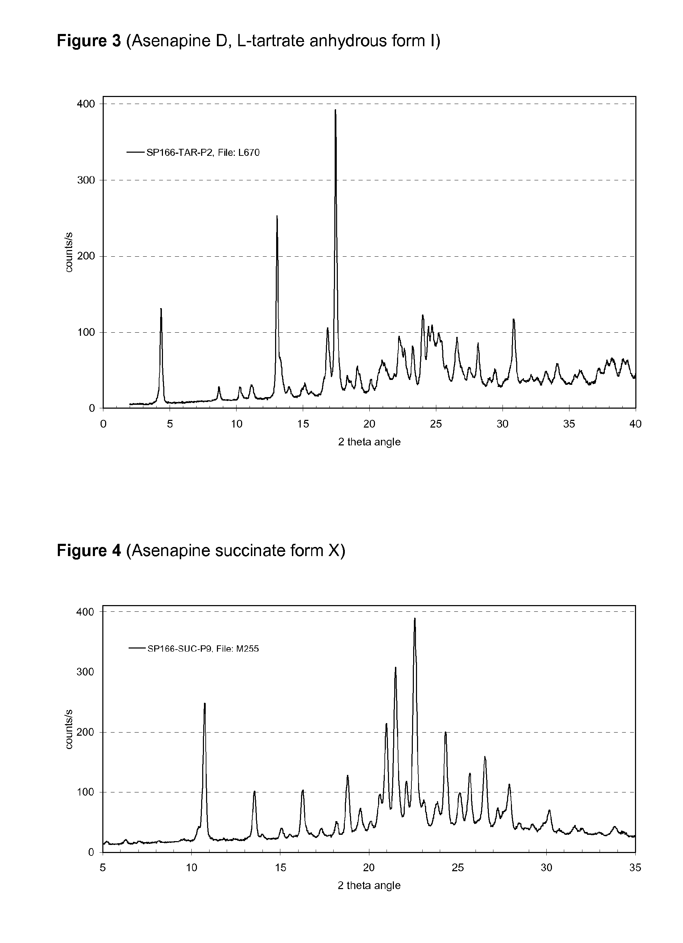

The crystalline Asenapine D, L-tartrate anhydrous form I is preferably characterized by a PXRD pattern substantially in accordance with Figure 3, in particular the PXRD pattern comprises the peaks as given in Table 1.

The crystalline Asenapine succinate form X is preferably characterized by a PXRD pattern substantially in accordance with Figure 4, in particular the PXRD pattern comprises the peaks as given in Table 8.

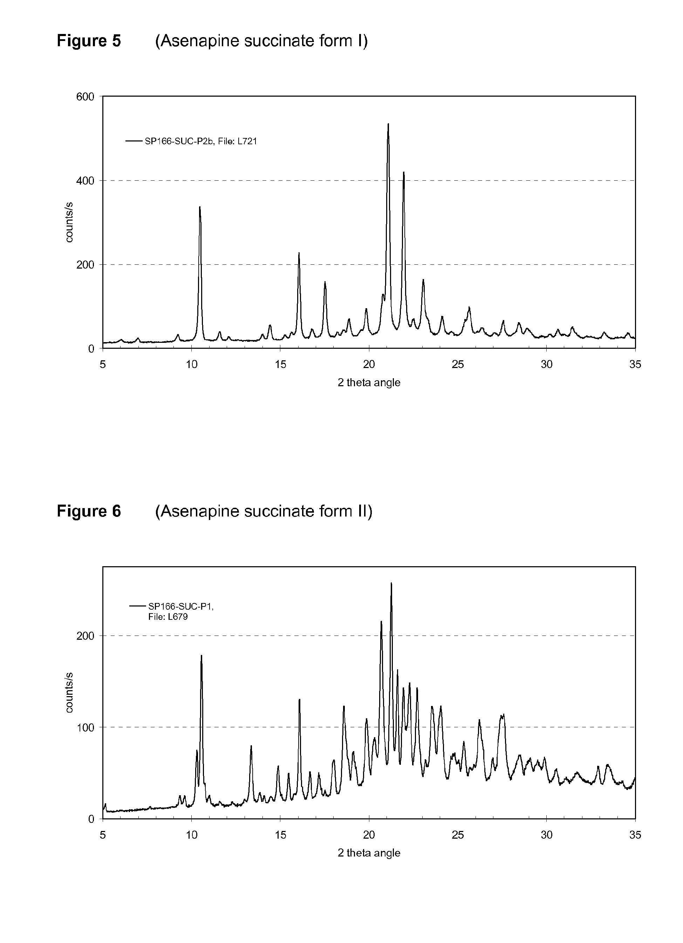

The crystalline Asenapine succinate form I is preferably characterized by a PXRD pattern substantially in accordance with Figure 5, in particular the PXRD pattern comprises the peaks as given in Table 6.

The crystalline Asenapine succinate form II is preferably characterized by a PXRD pattern substantially in accordance with Figure 6, in particular the PXRD pattern comprises the peaks as given in Table 5.

In general, the Asenapine salts described herein are preferably characterized by their PXRD pattern, i.e. the peaks as given in the respective peak tables. Particularly suitable for characterizing the salts are the peaks having an intensity of vs (very strong), s (strong) and m (medium).
(5) A process for preparing crystalline Asenapine salts according to any of items (1)-(4) comprising the steps of
   a) combining Asenapine free base with tartaric acid, preferably D, L-tartaric acid, or succinic acid in a solvent, optionally in the presence of seed crystals of the crystalline Asenapine salts, and
   b) recovering crystalline Asenapine salts.
(6) The process of item (5), wherein the obtained crystalline Asenapine salts are selected from the group consisting of: Asenapine D, L-tartrate anhydrous form I, Asenapine succinate form I, and Asenapine succinate form II.
(7) The process of item (5) or (6), wherein the solvent or solvent mixture comprises or consists of one or more organic solvents from the group consisting of C₂-C₄ alcohols, preferably ethanol or methanol, and acetic acid C₂-C₄ alkyl, preferably ethyl acetate, and optionally comprises water, preferably in an amount of up to 30%.
(8) A process for preparing crystalline Asenapine hydrates as defined in any of items (1)-(4) comprising the step of
   a) preparing a suspension of crystalline Asenapine salts obtainable or obtained by the process of any of items (5)-(7) or Asenapine salts as defined in items (1)-(4) in an aqueous solvent, preferably in water or a solvent mixture comprising water, and optionally in the presence of seed crystals, and
   b) isolating crystalline Asenapine hydrates from the suspension. In the aforementioned embodiment, the crystalline Asenapine salts used in step a) differ from the crystalline Asenapine hydrates that are prepared by the process and which are isolated in step b). Of course, it is only possible to convert Asenapine tartrates into different types of Asenapine tartrates and Asenapine succinates into different types of Asenapine succinates.
(9) The process of item (8), wherein the Asenapine salts used in step a) are selected from the group consisting of: Asenapine D, L-tartrate anhydrous form I, Asenapine D, L-tartrate hydrate form B, Asenapine succinate form I, and Asenapine succinate form II, and wherein the isolated crystalline Asenapine hydrates are preferably selected from the group consisting of: Asenapine D, L-tartrate hydrate form A, Asenapine D, L-tartrate hydrate form B, and Asenapine succinate form X. In the aforementioned embodiment, using Asenapine D, L-tartrate hydrate form B in step a) results in the formation of Asenapine D, L-tartrate hydrate form A in step b).
(10) A process for preparing crystalline Asenapine D, L-tartrate hydrate form A as defined in item (4), comprising
   a) preparing a suspension of Asenapine D, L-tartrate anhydrous form I as defined in item (4) or Asenapine D, L-tartrate hydrate form B as defined in item (4) in an aqueous solvent, preferably in water or a solvent mixture comprising water, optionally in the presence of seed crystals, and
   b) isolating crystalline Asenapine D, L-tartrate hydrate form A from the suspension.
(11) A process for preparing crystalline Asenapine succinate form X as defined in item (4), comprising
   a) preparing a suspension of Asenapine succinate form I as defined in item (4) or Asenapine succinate form II as defined in item (4), in an aqueous solvent, preferably in water or a solvent mixture comprising water, optionally in the presence of seed crystals, and
   b) isolating crystalline Asenapine succinate form X from the suspension.
(12) Crystalline salts of Asenapine with an organic acid according to formula I according to any of items (1)-(4) or as prepared according to any of items (5)-(11) for use as medicament.
(13) Crystalline salts of Asenapine according to any of items (1)-(4) or as prepared according to any of items (5)-(11) for use as medicament for psychotic diseases or disorders, wherein the salt preferably is Asenapine D, L-tartrate hydrate form A or Asenapine succinate form X.
(14) Pharmaceutical compositions comprising crystalline salts of Asenapine according to any of items (1)-(4) or crystalline salts of Asenapine prepared according to the process as defined in any of items (5)-(11).
(15) Pharmaceutical dosage forms comprising crystalline salts of Asenapine according to any of items (1)-(4) or crystalline salts of Asenapine prepared according to the process as defined in any of items (5)-(11).

### List of figures

- Figure 1:: X-ray powder diffraction pattern of Asenapine D, L-tartrate hydrate form A.
- Figure 2:: X-ray powder diffraction pattern of Asenapine D, L-tartrate hydrate form B.
- Figure 3:: X-ray powder diffraction pattern of Asenapine D, L-tartrate anhydrous form I.
- Figure 4:: X-ray powder diffraction pattern of Asenapine succinate form X.
- Figure 5:: X-ray powder diffraction pattern of Asenapine succinate form I.
- Figure 6:: X-ray powder diffraction pattern of Asenapine succinate form II.

### Detailed description

In accordance with the present invention there are provided novel salts of organic di-acids, preferably hydrates, of formula I 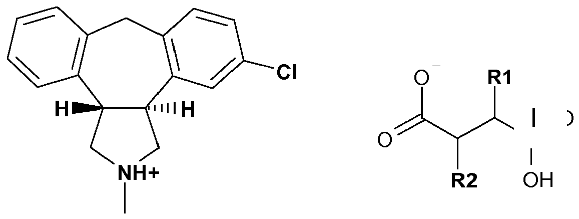 wherein R₁ and R₂ are the same and represent H or OH, preferably in substantially pure form. Preferably, the crystalline salts contain at least 90%, preferably at least 95%, further preferred at least 98% of one crystal polymorph as determined by PXRD analysis, wherein the PXRD peak of the desired crystalline polymorph having the highest intensity is compared with the peaks of the impurities.

The crystalline salts according to the invention preferably have a solubility in water of not less than 5 mg/ml, further preferred of not less than 6 mg/ml, even further preferred of not less than 8 mg/ml and most preferred of not less than 10 mg/ml calculated as free base equivalent. A typical maximum solubility can e.g. be 30 mg/ml, 20 mg/ml or 15 mg/ml The solubility of the salts is determined as described below.

Additionally preferred, the molar ratio of Asenapine to organic acid in said salt is about 1:1. The ratio of Asenapine to organic acid can also e.g. be 1:1.3 to 1.3:1. The ratio of Asenapine to organic acid can be determined by H-NMR spectroscopy and/or elementary analysis.

In accordance with the present invention the salts of Asenapine with organic di-acids as defined above comprise
a) crystalline succinate salts, and
b) crystalline tartrate salts.

More preferably, but not limited to, the tartrate salt is produced as the D, L-tartrate. The term "D, L-tartaric acid" or "D, L-tartrate" refers to an approximately 1:1 mixture of the levo (L) and dextro (D) forms. The use of the pure L tartrate is not preferred according to the present invention. Thus, in a first embodiment provided is a crystalline salt of Asenapine in form of a D, L-tartrate.

Within the meaning of the present invention, the term "seed crystals" refers to that type of crystals that shall be produced by the claimed method. For example, if it is desired to produce crystalline Asenapine D, L-tartrate hydrate form A, the seed crystals to be used to enhance the crystallization process are crystals of Asenapine D, L-tartrate hydrate form A.

Further preferred, the salts of the invention are selected from the group consisting of Asenapine D, L-tartrate hydrate form A, Asenapine D, L-tartrate hydrate form B, Asenapine succinate form X, Asenapine succinate form I, and Asenapine succinate form II. Also preferred, the salt is Asenapine D, L-tartrate hydrate form A or Asenapine succinate form X.

The present invention also refers to a process for preparing crystalline Asenapine salts according to the invention comprising the steps of
a) combining Asenapine free base with tartaric acid, preferably D, L-tartaric acid, or succinic acid in a solvent or mixture of solvents, optionally in the presence of seed crystals of said crystalline Asenapine salts and optionally in the presence of water, and
b) recovering crystalline Asenapine salts.

Preferably, the solvent or solvent mixture is chosen to be suitable for completely dissolving.

If tartaric or succinic acid as added in step a) is not completely dissolved in the solution containing Asenapine, it is preferred to heat the mixture in order to achieve complete dissolution. Preferably, the type and amount of solvent is chosen so that tartaric and succinic acid, respectively, only dissolve when increasing the temperature, preferably to the boiling temperature of the mixture. In order to enhance crystallization, it is preferred to then cool the solution and/or to concentrate the solution. However, it is also possible to add a solution of tartaric acid or succinic acid, e.g. in ethanol, to a solution of Asenapine, e.g. in ethanol.

Preferred solvents to be used in step a) and in the processes described herein in general include but are not limited to organic solvents or mixtures of organic solvents optionally including water. The organic solvents or mixtures of organic solvents comprise or consist of one or more solvents selected from C₂-C₄ alcohols, preferably ethanol and ethyl acetate, and C₂-C₄ alkyl esters, preferably acetic acid.

Particularly preferred combinations of solvents are methanol/acetic acid and ethanol/acetic acid.

An acetic acid C₂-C₄ alkyl ester can also be added as an antisolvent in step a). The solution of step a) is optionally concentrated and seeds of the Asenapine salts to be produced are optionally added to induce crystallization. Crystallization may then be completed by addition of the antisolvent as defined above. If it is desired to prepare crystalline salts in anhydrous form, the solvent or solvent mixture preferably does not contain water or only minor amounts of water of up to 5%, preferably up to 2%.

Preferably, the obtained crystalline Asenapine salts are selected from the group consisting of: Asenapine D, L-tartrate anhydrous form I, Asenapine succinate form I, and Asenapine succinate form II. If water is present in the solvent it is possible to directly obtain hydrates of Asenapine salts.

The amount of D, L-tartaric acid is not critical, e.g. 0.8 to 2.0 moles of tartaric acid/succinic acid per mole of Asenapine may be used. Preferably a stoichiometric amount or a slight excess, e.g. 1.0 to 1.2 mol of tartaric acid/succinic acid per mol of Asenapine base is used.

The present invention also refers to a process for preparing crystalline Asenapine hydrates according to the invention comprising the steps of
a) preparing a suspension of crystalline Asenapine salts according to formula I, preferably Asenapine salts obtainable or obtained by the above-described process in an aqueous solvent, preferably in water, and optionally in the presence of seed crystals, and
b) isolating crystalline Asenapine hydrates from the suspension.

Preferably, the mass weight ratio of crystalline Asenapine salt in step a) to solvent is 1/5 to 1/50, preferably 1 /10 to 1/40.

Preferably, the suspension prepared in step a) is kept at a temperature of between 20° to 30°C for a period of at least 2 days, preferably at least 3 days, prior to isolating the product.

Preferably, the Asenapine salts used in step a) of the process are selected from the group consisting of: Asenapine D, L-tartrate anhydrous form I, Asenapine D, L-tartrate hydrate form B, Asenapine succinate form I, and Asenapine succinate form II, and wherein the isolated crystalline Asenapine hydrates that are obtained in step b) are preferably selected from the group consisting of: Asenapine D, L-tartrate hydrate form A, Asenapine D, L-tartrate hydrate form B, and Asenapine succinate form X.

In one embodiment, the process comprises
a) preparing a suspension of Asenapine D, L-tartrate anhydrous form I or Asenapine D, L-tartrate hydrate form B in an aqueous solvent, preferably in water, optionally in the presence of seed crystals, and
b) isolating crystalline Asenapine D, L-tartrate hydrate form A from the suspension.

Preferred solvents and process conditions are described above.

In another embodiment, the process comprises
a) preparing a suspension of Asenapine succinate form I or Asenapine succinate form II, in an aqueous solvent, preferably in water, optionally in the presence of seeds crystals, and
b) isolating crystalline Asenapine succinate form X from the suspension.

Preferred solvents and process conditions are also described above.

In one embodiment, the present invention refers to crystalline Asenapine D, L-tartrate anhydrous form I. The crystalline Asenapine D, L-tartrate anhydrous form I of the invention is characterized by X-ray powder diffraction reflections comprising peaks at about 4.4°± 0.2°, 13.1°± 0.2°, 16.9°± 0.2°, 17.5°± 0.2° and 30.8° ± 0.2° degrees two-theta. The crystalline Asenapine D, L-tartrate anhydrous form I can be further characterized by a PXRD pattern substantially in accordance with Figure 3.

The H-NMR (nuclear magnetic resonance) spectrum and the elementary analysis confirmed the expected 1:1 ratio of Asenapine to tartaric acid. Upon heating to 180°C at a rate of 10 K/min the thermogravimetric analysis of the crystalline Asenapine D, L-tartrate anhydrous form I showed basically no mass loss, e.g. about 0.1 %.

Crystalline Asenapine D, L-tartrate anhydrous form I may be prepared by
a) combining Asenapine free base with D, L-tartaric acid in a suitable solvent, and
b) recovering crystalline Asenapine D, L-tartrate anhydrous form I from the suspension.

Preferred solvents and process conditions are also described above.

Preferred solvents also include but are not limited to mixtures of C₂-C₄alcohols and acetic acid C₂-C₄ alkyl esters, preferably ethanol and ethyl acetate. Preferably

Asenapine base is dissolved in ethanol and combined with a solution of D, L-tartaric acid in ethanol. An acetic acid C₂-C₄ alkyl ester is added as an antisolvent. The solution is optionally concentrated and seeds of Asenapine D, L-tartrate anhydrous form I are optionally added to induce crystallization. Crystallization may then be completed by addition of the antisolvent as defined above.

The amount of D, L-tartaric acid is not critical, e.g. 0.8 to 2.0 moles of tartaric acid per mole of Asenapine may be used. Preferably, a stoichiometric amount or a slight excess, e.g. 1.0 to 1.2 mol of tartaric acid per mol of Asenapine base are used.

In an aspect of the invention there is provided a crystalline Asenapine D, L-tartrate hydrate form A. The crystalline Asenapine D, L-tartrate hydrate form A of the invention is characterized by X-ray powder diffraction reflections comprising peaks at about 13.3° ± 0.2°, 15.0 ± 0.2°, 17.4° ± 0.2°, 18.5° ± 0.2°, 24.4° ± 0.2° and 26.6° ± 0.2° degrees two-theta. The crystalline D, L-tartrate hydrate form A of the invention can be further characterized by a PXRD pattern substantially in accordance with Figure 1.

Form A of the crystalline D, L-tartrate of Asenapine is obtained by suspending Asenapine D, L-tartrate hydrate form B as described below or the crystalline Asenapine D, L-tartrate anhydrous form I in water, optionally in the presence of seeds of Form A, e.g. by stirring a suspension of the crystalline Asenapine D, L-tartrate anhydrous form I or Asenapine D, L-tartrate hydrate form B in an aqueous solvent, preferably in water, at e.g. about 25°C for several hours or days, e.g. 1 to 2 days, more preferably for about 3 days, optionally in the presence of seeds of D, L-tartrate form A.

Preferred solvents and process conditions are also described above.

Seeds of crystalline Asenapine D, L-tartrate hydrate form A can e.g. be obtained by stirring Asenapine D, L-tartrate anhydrous form I in water at about 25°C for at least 3 days. Asenapine D, L-tartrate hydrate form A may optionally be recovered from the suspension by filtration and drying, e.g. by drying in vacuo.

In another aspect of the invention there is provided a crystalline Asenapine D, L-tartrate hydrate form B. The crystalline Asenapine D, L-tartrate hydrate form B of the invention is characterized by X-ray powder diffraction reflections comprising peaks at about 7.3°± 0.2°, 10.5°± 0.2°, 14.6°± 0.2°, 15.8°± 0.2°, 20.3°± 0.2°, 23.5°± 0.2° and 26.5°± 0.2° degrees two-theta. The crystalline Asenapine D, L-tartrate hydrate form B can be further characterized by a PXRD pattern substantially in accordance with Figure 2.

The aqueous solubility of the Asenapine D, L-tartrate hydrate form B was determined to be 11.5 mg/ml at 25°C calculated as free base whereas for the maleate it is mentioned in Funke et. al. (Arzneim.-Forsch./Drug Res. 40, 1999, 536-539) that a saturated solution at 23°C has a concentration of 5.8 mg/ml at pH = 4.4. This translates into a free base solubility of about 4.1 mg/ml.

The aqueous solubility was determined after 48 hours of suspension equilibration at 25°C. Thereafter, the suspensions were filtered and the concentration in the liquid phase was determined by HPLC.

Crystalline Asenapine D, L-tartrate hydrate form B may be obtained by stirring Asenapine D, L-tartrate anhydrous form I in water for a time sufficient to convert Asenapine D, L-tartrate anhydrous form I to crystalline Asenapine D, L-tartrate hydrate form B, e.g. by stirring Asenapine D, L-tartrate anhydrous form I in water at about 25°C for about 2 days. Asenapine D, L-tartrate form B may optionally be recovered from the suspension by filtration and drying, e.g. by drying in vacuo.

In a second embodiment provided is a crystalline salt of Asenapine in form of a succinate.

In an aspect of the invention there is provides a crystalline Asenapine succinate hydrate form X.

The crystalline Asenapine succinate hydrate form X of the invention is characterized by X-ray powder diffraction reflections comprising peaks at about 10.8°± 0.2°, 21.0°± 0.2°, 21.5°± 0.2°, 22.6°± 0.2° and 23.4° ± 0.2° degrees two-theta. The crystalline Asenapine succinate hydrate form X of the invention can be further characterized by a PXRD pattern substantially in accordance with Figure 4.

The crystalline Asenapine succinate hydrate form X may contain about 1% to 5% of water.

The crystalline Asenapine succinate hydrate form X is prepared by preparing a suspension of Asenapine succinate form I as described below or Asenapine succinate form II as described below in an aqueous solvent, preferably in water, optionally in the presence of seed crystals, for a sufficient time to induce complete formation of form X, e.g. for a period of several hours to several days, e.g. for about 2 days, e.g. at ambient temperate, e.g. at about 20°C to 30°C, e.g. at about 25°C. The aqueous solubility of the Asenapine succinate form X of the invention was determined to be 10.7 mg/ml at 25°C calculated as free base.

In another aspect of the invention there is provided a crystalline Asenapine succinate hemihydrate, designated as form I. The crystalline Asenapine succinate form I is characterized by X-ray powder diffraction reflections comprising peaks at about 10.5°± 0.2°, 16.5°± 0.2°, 21.1°± 0.2°, 22.0°± 0.2° and 23.0° ± 0.2° degrees two-theta. Asenapine succinate form I can be further characterized by a PXRD pattern substantially in accordance with Figure 5.

The H-NMR spectrum complies with a 1:1 salt of Asenapine and succinic acid.

The hygroscopic nature of the succinate salt hemihydrate was investigated by dynamic vapor sorption. At relative humidities greater than about 20% the investigated solid form of the succinate can contain about 2 to 2.5 % of water. This is in agreement with the results of the elemental analysis and suggests that above this relative humidity solid state form is a hemihydrate.

The Asenapine succinate form I of the invention may be prepared by
a) combining Asenapine free base with succinic acid in a suitable solvent or solvent mixture, and
b) crystallizing Asenapine succinate form I, optionally in the presence of seed crystals.

Preferred solvents and process conditions are also described above.

Preferred solvents or solvent mixtures are indicated above and also include but are not limited to mixtures of C₂-C₄ alcohols and acetic acid C₂-C₄ alkyl esters, preferably ethanol and ethyl acetate. Preferably Asenapine base is dissolved in ethanol and combined with a solution of succinic acid in ethanol. An acetic acid C₂-C₄ alkyl ester is added as an antisolvent. The solution is optionally concentrated and seeds of Asenapine form I are optionally added to induce crystallization. Crystallization may then be completed by addition of the antisolvent as defined above. The Asenapine succinate form I may be then isolated by standard procedures, e.g. by filtration and drying, e.g. drying in vacuo.

Alternatively succinic acid may be used as such. The amount of succinic acid is not critical, e.g. 0.8 to 2.0 moles of succinic acid per mole of Asenapine may be used. Preferably a stoichiometric amount or a slight excess, e.g. 1.0 to 1.2 mol of succinic acid per mol of Asenapine base are used.

In another embodiment provided is a crystalline Asenapine succinate designated as form II. The crystalline Asenapine succinate (ratio of Asenapine/succinate of about 1 : 1) form II is characterized by X-ray powder diffraction reflections comprising peaks at about 10.6°± 0.2°, 16.1°± 0.2°, 20.7°± 0.2°, 21.2°± 0.2° and 22.7° ± 0.2° degrees two-theta. Asenapine succinate form II can be further characterized by a PXRD pattern substantially in accordance with Figure 6.

The Asenapine succinate form II of the invention may be prepared by
a) combining Asenapine free base with succinic acid in a suitable solvent or solvent mixture, and
b) crystallizing Asenapine form II, optionally in the presence of seed crystals.

Preferred solvents and process conditions are also described above.

Preferred solvents include but are not limited to ethyl acetate or mixtures of methanol and ethyl acetate. For example Asenapine base is dissolved in ethyl acetate and the solution is combined with a solution of succinic acid in methanol. An acetic acid C₂-C₄ alkyl ester is added as an antisolvent. The solution is concentrated and to the concentrate ethyl acetate is added. Crystallization may then be completed by stirring the suspension e.g. by stirring the suspension for several hours e.g. by stirring for 24 hours. The Asenapine succinate form II may then be isolated by standard procedures, e.g. by filtration and drying, e.g. drying in vacuo.

The amount of succinic acid used for the crystallization of Asenapine succinate form II is preferably a stoichiometric amount or a slight excess, e.g. 1.0 to 1.2 mol of succinic acid per mol of Asenapine base.

The crystalline salts of Asenapine according to the present invention can be used as medicaments and for the manufacture of medicaments, respectively. In particular, the crystalline salts of Asenapine can be used as medicament for psychotic diseases or disorders, wherein the salt preferably is Asenapine D, L-tartrate hydrate form A or Asenapine succinate form X.

The present invention also refers to pharmaceutical compositions comprising crystalline salts of Asenapine according to the present invention.

Another embodiment of the present invention is a pharmaceutical formulation or dosage form comprising one or more Asenapine salts of the present invention and at least one pharmaceutically acceptable carrier or diluent.

Preferred salts of the invention to be used in the pharmaceutical compositions, pharmaceutical formulations and dosage forms, respectively, are described above, wherein the salts are preferably selected from the group consisting of Asenapine D, L-tartrate hydrate form A, Asenapine D, L-tartrate hydrate form B, Asenapine succinate form X, Asenapine succinate form I, and Asenapine succinate form II. Also preferred, the salt is Asenapine D, L-tartrate hydrate form A or Asenapine succinate form X.

The novel crystalline Asenapine salts of the invention may be formulated by using any known techniques, for example as disclosed in example 16 of WO 2006/106135 by mixing the novel crystalline salts into a gelatin/mannitol/water mixture and freeze drying, preferably after dosing into pre-formed pockets.

Powder X-ray diffraction: PXRD was carried out with a Bruker D8 Advance powder X-ray diffractometer using Cu_{Kα} radiation in reflection (Bragg-Brenatno) geometry. 2θ values usually are accurate within an error of ±0.1-0.2°. The samples were generally prepared without any special treatment other than the application of slight pressure to get a flat surface. Two different silicon single crystal sample holder types were used: a) a standard holder with 0.1 mm in depth, and b) a 0.5 mm depth sample holder with 12 mm cavity diameter. Normally samples were measured uncovered. The tube voltage was 40 kV and current was 40 mA. The PXRD diffractometer is equipped with a LynxEye detector. A variable divergence slight was used with a 3° window. The step size was 0.02° 2θ with a step time of 37 seconds. The samples were rotated at 0.5 rps during the measurement.

### TG-FTIR:

Thermogravimetric measurements were carried out with a Netzsch Thermo-Microbalance TG 209 coupled to a Bruker FTIR Spectrometer Vector 22 (sample pans with a pinhole, N₂ atmosphere, heating rate 10 K/min).

### Solvents:

For all experiments, Fluka or Merck analytical grade solvents were used.

### HPLC method:

An HPLC instrument from TSP (UV3000, AS3000, P4000, SCM1000 using software version 4.1) was used in combination with a column from Waters (XTerra MS C18, 4.6 x 100 mm, 5 µm (CC01A)). The mobile phase A was H₂O / ACN 95:5 with 0.1 % TFA, and mobile phase B was H₂O / ACN 5:95 with 0.1 % TFA. The reference concentration was 0.09 mg/mL. The Asenapine peak appeared at a retention time.

4.0 - 4.1 min. The method was isocratic with 70% mobile phase A and 30% mobile phase B at 0 min and after 10 min at a flow of 1.0 mL/min. The injection volume was 10 µL and the detection wavelength 202 nm.

### Determining solubility of Asenapine salts:

The aqueous solubility of Asenapine salts is determined in water after 48 hours of suspension equilibration at 25°C. Thereafter, the suspension is filtered and the concentration in the liquid phase was determined by HPLC as described above.

### Reference example:

6.43 g of Asenapine maleate are added with stirring to a mixture of 40 ml of 1 m NaOH and 320 ml of methyl- tert. butyl ether. The mixture is stirred for 10 min and the layers are separated. The aqueous layer is extracted with 320 ml of methyl-tert. butyl ether. The combined organic layers are extracted with 150 ml of water.

The organic layer is dried with 13.6 g of sodium sulfate. The suspension is filtered and the solution is concentrated in vacuo at about 40°C and 20 ml.

### Yield:

3.77 g of Asenapine free base (as an oil)

### Example 1

Preparation of the Asenapine D, L-tartrate anhydrous form I seed crystals 150 mg of Asenapine free base (in form of an oil containing small amounts of residual solvents, i.e., ∼ 0.5 mmol) is dissolved in 2.0 ml ethanol and 78 mg of D,L tartaric acid (Fluka # 95330) is added. In order to dissolve the tartaric acid the mixture is heated to boiling temperature. After letting cool to room temperature the mixture is stirred for about one hour, if no immediate crystallization is observed the solvent is slowly evaporated under nitrogen at a flow rate of about 30 ml/min.

After evaporation of almost all solvent the evaporation is stopped and 2.0 ml ethyl acetate is added. After overnight stirring a suspension is obtained, if the viscosity of the suspension is high, about 0.5 ml acetonitrile is added, then the suspension is filtered and the vial is washed out with 3 ml of methyl- tert. butyl ether. The obtained solid is dried under vacuum overnight at room temperature, then powder X-ray diffraction, H-NMR spectroscopy, TG-FTIR, and light microscopy is performed. The H-NMR spectrum complies with an Asenapine tartrate salt with a 1:1 stoichiometry. TG-FTIR indicates that an essentially solvent-free solid form is obtained. The PXRD pattern as shown in Figure 3 of which the most important peaks are listed in Table 1 shows that the salt is Asenapine D, L-tartrate anhydrous form I without any significant amounts of amorphous form. The yield is about 170 mg.

**Table 1: PXRD peak table for Asenapine D, L-tartrate anhydrous form 1.**

| Angle 2θ | d-spacings [Ǻ] | qualitative relative intensity |
|---|---|---|
| 4.4 | 20.22 | m |
| 8.7 | 10.15 | w |
| 10.3 | 8.60 | w |
| 11.1 | 7.94 | w |
| 13.1 | 6.77 | s |
| 16.9 | 5.25 | m |
| 17.5 | 5.07 | vs |
| 21.0 | 4.23 | m |
| 21.4 | 4.16 | w |
| 22.2 | 3.99 | m |
| 22.6 | 3.93 | m |
| 23.3 | 3.82 | m |
| 24.0 | 3.71 | m |
| 24.4 | 3.64 | m |
| 24.7 | 3.60 | m |
| 25.2 | 3.53 | m |
| 25.4 | 3.51 | m |
| 26.6 | 3.35 | m |
| 27.5 | 3.24 | w |
| 28.1 | 3.17 | m |
| 30.8 | 2.90 | s |
| 34.1 | 2.63 | w |
| 37.8 | 2.38 | w |
| 38.2 | 2.35 | m |
| 39.0 | 2.31 | m |
| 39.4 | 2.29 | m |

### Example 2

Preparation of the Asenapine D, L-tartrate anhydrous form I 300 mg of Asenapine free base (in form of an oil containing small amounts of residual solvents, i.e., ∼ 1 mmol) is dissolved in 3.0 ml ethanol and to this solution is added 154 mg D, L-tartaric acid (Fluka # 95330). Dissolution of the tartaric acid is achieved by shortly heating to boiling temperature and after letting the solution cool to room temperature about 2 mg of Asenapine D, L-tartrate seed crystals according to example 1 is added and a suspension slowly begins to form. 3.0 ml of ethyl acetate is added to reduce the solubility and stirring is continued for about three hours, then 4.0 ml additional ethyl acetate is added and stirring continued overnight at room temperature. The solid is filtered off and dried under vacuum at room temperature for about 20 hours. Powder X-ray diffraction shows that the same crystalline form I of the Asenapine D, L-tartrate is obtained. The results of the elemental composition analysis is given in Table 2 confirm that an anhydrous Asenapine D, L-tartate is obtained.

**Table 2: Result of the elemental composition analysis for crystalline Asenapine D, L-tartrate anhydrous form 1:**

| Element | % Found |
|---|---|
| C | 57.9 |
| H | 5.2 |
| N | 3.4 |
| O | 26.0 |
| Cl | 8.1 |

### Example 3

Preparation of the Asenapine D, L-tartrate hydrate form B 87 mg of Asenapine D, L-tartrate form I according to Example 2 is suspended in 2.0 ml water and placed on a laboratory shaker at 25°C and 600 rpm for two days. After two days of equilibration the suspension is filtered and the recovered solid investigated by PXRD. The obtained PXRD pattern corresponds to the crystalline form B as depicted in Figure 2 with peak locations as provided in Table 3. The aqueous solubility is determined by measuring the concentration of Asenapine in the filtered solution by HPLC. Karl Fischer titration reveals water content of about 4.8%.

**Table 3: PXRD peak table for the Asenapine D, L-tartrate hydrate form B.**

| Angle 2θ | d-spacings [Ǻ] | qualitative relative intensity |
|---|---|---|
| 7.3 | 12.14 | s |
| 7.6 | 11.55 | m |
| 10.5 | 8.43 | s |
| 10.9 | 8.11 | w |
| 14.1 | 6.30 | m |
| 14.4 | 6.16 | m |
| 14.6 | 6.05 | s |
| 15.4 | 5.77 | m |
| 15.8 | 5.61 | s |
| 17.2 | 5.15 | w |
| 18.3 | 4.86 | m |
| 19.8 | 4.48 | w |
| 20.3 | 4.38 | s |
| 20.5 | 4.32 | m |
| 21.0 | 4.23 | w |
| 21.2 | 4.19 | w |
| 21.6 | 4.12 | w |
| 22.0 | 4.03 | m |
| 23.1 | 3.85 | w |
| 23.5 | 3.78 | s |
| 24.3 | 3.66 | w |
| 24.6 | 3.62 | w |
| 25.3 | 3.52 | w |
| 26.5 | 3.36 | vs |
| 37.5 | 2.40 | m |

### Example 4

Preparation of the crystalline Asenapine D, L-tartrate form I Dissolve 513 mg of Asenapine free base (in form of an oil containing small amounts of residual solvents, i.e., ∼ 1.75 mmol) and 257 mg of D, L-tartaric acid in 10.0 ml ethanol under heating to boiling temperature. Let clear solution cool to room temperature and evaporate about 4 ml of solvent under nitrogen (at a flow rate of about 10 ml/min) then add 7.0 ml ethyl acetate. Stirring is continued overnight at room temperature before the suspension is filtered and the solid product dried under vacuum at room temperature for about 20 hours. The yield is about 600 mg. Powder X-ray diffraction shows that the crystalline form I of the Asenapine D, L-tartrate is obtained.

### Example 5

Preparation of the crystalline Asenapine D, L-tartrate form A seeds To 100 mg of Asenapine D, L-tartrate form I according to example 2 1.0 ml water is added and the resulting suspension stirred for three days at room temperature, then the solid is separated by filtration and after short drying in air at r.t. investigated by PXRD. The PXRD pattern as shown in Figure 1 with peak locations as provided in Table 4 is associated to form A of Asenapine D, L-tartrate hydrate. The H-NMR confirms the chemical integrity of the compound and a 1:1 salt stoichiometry. Karl Fischer titration reveals water content of about 7%.

**Table 4: PXRD peak table for the Asenapine D, L-tartrate hydrate form A.**

| Angle 2θ | d-spacings [Ǻ] | qualitative relative intensity |
|---|---|---|
| 6.6 | 13.29 | w |
| 9.2 | 9.59 | w |
| 10.8 | 8.20 | w |
| 13.3 | 6.66 | s |
| 14.2 | 6.23 | m |
| 15.0 | 5.88 | m |
| 17.4 | 5.09 | s |
| 18.5 | 4.80 | vs |
| 20.0 | 4.44 | m |
| 20.2 | 4.39 | m |
| 21.6 | 4.12 | w |
| 22.5 | 3.95 | w |
| 22.7 | 3.92 | m |
| 22.9 | 3.89 | w |
| 23.1 | 3.85 | w |
| 23.5 | 3.78 | m |
| 24.4 | 3.64 | vs |
| 24.6 | 3.61 | m |
| 26.6 | 3.34 | m |
| 26.6 | 3.33 | m |
| 27.0 | 3.30 | m |
| 27.6 | 3.23 | m |
| 28.6 | 3.12 | w |
| 29.0 | 3.08 | m |
| 29.4 | 3.03 | w |
| 31.2 | 2.86 | w |
| 31.4 | 2.85 | w |
| 33.3 | 2.69 | w |
| 33.6 | 2.66 | m |
| 37.4 | 2.40 | w |

### Example 6

Preparation of Asenapine D, L-tartrate hydrate form A To about 200 mg of Asenapine D, L-tartrate form I is added 2.0 ml water and suspension stirred for about four hours. Then the suspension is seeded with Asenapine D, L-tartrate hydrate form A and stirring is continued for three days at r.t. The suspension is filtered and the obtained crystalline form investigated by powder X-ray diffraction, which confirms that Asenapine D, L-tartrate hydrate form A with a PXRD pattern as shown in Figure 1 is obtained. Further analysis by Karl Fischer titration indicated a water content of about 7%.

### Example 7

Preparation of the crystalline Asenapine succinate seed crystals form II 100 mg of Asenapine free base (in form of an oil containing small amounts of residual solvents, i.e., ∼ 0.35 mmol) is dissolved in 3.0 ml ethyl acetate and succinic acid (Fluka #14079) is added in form of a 1 M stock solution in methanol (41.2 mg in 0.66 ml. A clear solution is obtained from which the solvents are evaporated under nitrogen. To the oily residue after evaporation 1.0 ml ethyl acetate is added and after overnight stirring at room temperature a suspension with a crystalline product is obtained. The obtained PXRD pattern which is shown in Figure 6 and for which the peak locations are listed in Table 5 corresponds to Asenapine succinate form II.

**Table 5: PXRD peak table for Asenapine succinate form II.**

| Angle 2θ | d-spacings [Ǻ] | qualitative relative intensity |
|---|---|---|
| 10.3 | 8.59 | m |
| 10.6 | 8.36 | s |
| 13.4 | 6.62 | m |
| 14.9 | 5.95 | m |
| 16.1 | 5.51 | s |
| 18.0 | 4.92 | m |
| 18.6 | 4.77 | s |
| 18.7 | 4.74 | s |
| 18.8 | 4.71 | m |
| 19.1 | 4.64 | m |
| 19.3 | 4.61 | m |
| 19.8 | 4.47 | s |
| 20.3 | 4.37 | s |
| 20.7 | 4.29 | vs |
| 21.2 | 4.18 | vs |
| 21.6 | 4.11 | s |
| 21.9 | 4.05 | s |
| 22.3 | 3.99 | s |
| 22.7 | 3.91 | s |
| 23.2 | 3.83 | m |
| 23.5 | 3.78 | s |
| 24.0 | 3.70 | s |
| 25.3 | 3.51 | s |
| 26.2 | 3.40 | s |
| 26.4 | 3.37 | s |
| 27.3 | 3.27 | s |
| 27.4 | 3.25 | s |
| 27.6 | 3.23 | s |
| 28.5 | 3.13 | m |
| 33.4 | 2.68 | m |

### Example 8

Preparation of the crystalline Asenapine succinate form I (hemihydrate) 500 mg of Asenapine free base (in form of an oil containing small amounts of residual solvents, i.e., ∼ 1.7 mmol) is dissolved in 4.0 ml ethanol and 182 mg succinic acid (solid, Fluka #14079) is added then the succinic acid is also dissolved by shortly heating to boiling temperature. Let cool to room temperature and then 7.0 ml ethyl acetate is added and the solvents are slowly evaporated under a slight nitrogen flow of about 10 ml/min to a remaining volume of about 2.5 ml. The solution is seeded with crystalline succinate salt according to Example 9 and upon further stirring a suspension with crystalline material is obtained. 3.0 ml of ethyl acetate is added and stirring is continued at room temperature for three days before the solid is separated by filtration. After drying under vacuum at 40°C for about 20 hours about 280 mg of crystalline Asenapine succinate salt is obtained, which is investigated by powder X-ray diffraction, elemental composition analysis, H-NMR spectroscopy, light microscopy and TG-FTIR. The PXRD pattern is displayed in Figure 5 and the corresponding peaks list is provided in Table 6. TG-FTIR shows a small mass loss of about 2% which is attributable to water and the elemental composition analysis is consistent with an Asenapine succinate salt that contains about 0.5 equivalents of water as indicated in Table 7.

**Table 6: PXRD peak table for the Asenapine succinate form I (hemihydrate)**

| Angle 2θ | d-spacings [Ǻ] | qualitative relative intensity |
|---|---|---|
| 10.5 | 8.44 | s |
| 16.5 | 5.52 | s |
| 17.5 | 5.06 | m |
| 18.9 | 4.70 | w |
| 19.8 | 4.47 | m |
| 20.8 | 4.28 | m |
| 21.1 | 4.21 | vs |
| 22.0 | 4.05 | vs |
| 22.5 | 3.95 | w |
| 23.0 | 3.86 | s |
| 24.1 | 3.69 | m |
| 25.4 | 3.50 | m |
| 25.6 | 3.47 | m |
| 26.4 | 3.38 | w |
| 27.5 | 3.24 | w |
| 28.4 | 3.14 | w |
| 28.9 | 3.09 | w |
| 30.1 | 2.96 | w |
| 30.6 | 2.91 | w |
| 31.4 | 2.84 | w |
| 33.2 | 2.69 | w |

**Table 7: Result of the elemental composition analysis for the crystalline Asenapine succinate form I (hemihydrate)**

| Element | % Found |
|---|---|
| C | 60.7 |
| H | 5.8 |
| N | 3.6 |
| O | 21.5 |
| Cl | 8.5 |

### Example 9

Preparation of the crystalline Asenapine succinate salt form X To 89 mg of Asenapine succinate form I is added 1.0 ml water and the obtained suspension is stirred at 25°C. After two days of equilibration the suspension is filtered and the recovered solid investigated by PXRD. The obtained PXRD pattern corresponds to the crystalline Asenapine succinate form X as depicted in Figure 4 with peak locations as provided in Table 8. The aqueous solubility was determined by measuring the concentration of Asenapine in the filtered solution by HPLC.

**Table 8: PXRD peak table for the Asenapine succinate form X**

| Angle 2θ | d-spacings [Ǻ] | qualitative relative intensity |
|---|---|---|
| 6.3 | 14.0 | vw |
| 10.8 | 8.2 | vs |
| 13.5 | 6.5 | m |
| 15.1 | 5.86 | vw |
| 16.3 | 5.44 | m |
| 17.3 | 5.12 | vw |
| 18.2 | 4.88 | vw |
| 18.8 | 4.72 | m |
| 19.5 | 4.55 | w |
| 20.1 | 4.42 | vw |
| 20.6 | 4.31 | w |
| 21.0 | 4.23 | s |
| 21.5 | 4.13 | vs |
| 22.1 | 4.02 | m |
| 22.6 | 3.94 | vs |
| 23.2 | 3.84 | w |
| 23.8 | 3.73 | w |
| 24.3 | 3.66 | s |

## Claims

1. A crystalline salt, preferably a hydrate, of Asenapine with an organic acid
according to formula I 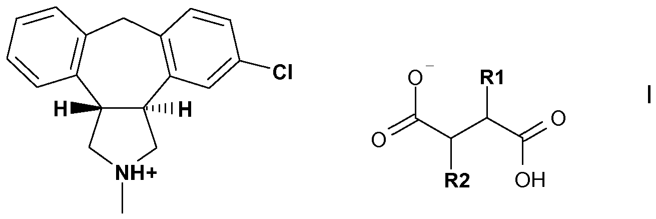 wherein R1 and R2 are the same and each represent H or OH.

2. The crystalline salt of claim 1 having a solubility in water of not less than 5 mg/ml calculated as free base equivalent and/or wherein the molar ratio of Asenapine to organic acid in said salt is between 1:1.3 to 1.3:1.

3. The crystalline salt of claim 1 or 2, wherein the organic acid represents D, L-tartaric acid or succinic acid.

4. The crystalline salt of any of claims 1-3, wherein said salt is selected from the group consisting of (i) Asenapine D, L-tartrate hydrate form A, (ii) Asenapine D, L-tartrate hydrate form B, (iii) Asenapine D, L-tartrate anhydrous form I, (iv) Asenapine succinate form X, (v) Asenapine succinate form I, and (vi) Asenapine succinate form II, wherein
(i) Asenapine D, L-tartrate hydrate form A is **characterized by** X-ray powder diffraction reflections (Cu Kα radiation) comprising peaks at about 18.5° ± 0.2°, 24.4°± 0.2°, 17.4° ± 0.2°, 13.3° ± 0.2°, 15.0° ± 0.2°, and 6.6° ± 0.2° degrees two-theta,
(ii) Asenapine D, L-tartrate hydrate form B **characterized by** X-ray powder diffraction reflections (Cu Kα radiation) comprising peaks at about 26.5°± 0.2°, 15.8°± 0.2°, 7.3°± 0.2°, 10.5°± 0.2°, 14.6°± 0.2°, 20.3°± 0.2°, and 23.5°± 0.2° degrees two-theta,
(iii) Asenapine D, L-tartrate anhydrous form I is **characterized by** X-ray powder diffraction reflections (Cu Kα radiation) comprising peaks at about 17.5°± 0.2°, 13.1°± 0.2°, 4.4°± 0.2°, 30.8° ± 0.2°, and 16.9°± 0.2° degrees two-theta,
(iv) Asenapine succinate form X is **characterized by** X-ray powder diffraction reflections (Cu Kα radiation) comprising peaks at about 22.6°± 0.2°, 21.5°± 0.2°, 10.8°± 0.2°, 21.0°± 0.2°, and 24.3° ± 0.2° degrees two-theta,
(v) Asenapine succinate form I is **characterized by** X-ray powder diffraction reflections (Cu Kα radiation) comprising peaks at about 21.1°± 0.2°, 22.0°± 0.2°, 10.5°± 0.2°, 16.5°± 0.2°, and 23.0° ± 0.2° degrees two-theta, and
(vi) Asenapine succinate form II is **characterized by** X-ray powder diffraction reflections (Cu Kα radiation) comprising peaks at about 21.2°± 0.2°, 20.7°± 0.2°, 10.6°± 0.2°, 22.7° ± 0.2°, and 16.1°± 0.2°, degrees two-theta.

5. A process for preparing crystalline Asenapine salts according to any of claims 1-4 comprising the steps of
a) combining Asenapine free base with tartaric acid, preferably D, L-tartaric acid, or succinic acid in a solvent or solvent mixture, optionally in the presence of seed crystals of said crystalline Asenapine salts, and
b) recovering crystalline Asenapine salts.

6. The process of claim 5, wherein the crystalline Asenapine salts obtained in step b) are selected from the group consisting of: Asenapine D, L-tartrate anhydrous form I, Asenapine succinate form I, and Asenapine succinate form II.

7. The process of claim 5 or 6, wherein the solvent or solvent mixture comprises one or more organic solvents from the group consisting of C₂-C₄ alcohols, preferably ethanol or methanol, and acetic acid C₂-C₄ alkyl esters, preferably ethyl acetate, and optionally comprises.

8. A process for preparing crystalline Asenapine hydrates according to any of claims 1-4 comprising the steps of
a) preparing a suspension of crystalline Asenapine salts obtainable or obtained by the process according to any of claims 5-7 or crystalline Asenapine salts according to claims 1-4 in an aqueous solvent or solvent mixture, preferably in water, and optionally in the presence of seed crystals, and
b) isolating crystalline Asenapine hydrates from the suspension.

9. The process of claim 8, wherein the Asenapine salts in step a) are selected from the group consisting of: Asenapine D, L-tartrate anhydrous form I, Asenapine D, L-tartrate hydrate form B, Asenapine succinate form I, and Asenapine succinate form II, and wherein the isolated crystalline Asenapine hydrates are preferably selected from the group consisting of: Asenapine D, L-tartrate hydrate form A, Asenapine D, L-tartrate hydrate form B, and Asenapine succinate form X.

10. The process of claim 9 for preparing crystalline Asenapine D, L-tartrate hydrate form A according to claim 4, comprising
a) preparing a suspension of Asenapine D, L-tartrate anhydrous form I according to claim 4 or Asenapine D, L-tartrate hydrate form B according to claim 4 in an aqueous solvent, preferably in water, optionally in the presence of seeds crystals, and
b) isolating crystalline Asenapine D, L-tartrate hydrate form A from the suspension.

11. The process of claim 9 for preparing crystalline Asenapine succinate form X according to claim 4, comprising
a) preparing a suspension of Asenapine succinate form I according to claim 4 or Asenapine succinate form II according to claim 4, in an aqueous solvent, preferably in water, optionally in the presence of seeds crystals, and
b) isolating crystalline Asenapine succinate form X from the suspension.

12. Crystalline salts, in particular hydrates, of Asenapine according to any of claims 1-4 or as prepared according to any of claims 5-11 for use as medicament.

13. Crystalline salts, in particular hydrates, of Asenapine according to any of claims 1-4 or as prepared according to any of claims 5-11 for use as medicament for psychotic diseases or disorders, wherein the salt preferably is Asenapine D, L-tartrate hydrate form A or Asenapine succinate form X.

14. Pharmaceutical composition comprising one or more crystalline salts, in particular hydrates, of Asenapine according to any of claims 1-4 or crystalline salts of Asenapine prepared according to the process of any of claims 5-11.

15. Pharmaceutical dosage form comprising one or more crystalline salts, in particular hydrates, of Asenapine according to any of claims 1-4 or crystalline salts of Asenapine prepared according to the process of any of claims 5-11.
